# EUROPEAN PATENT SPECIFICATION

(11) **EP 4 594 295 B1**
(45) Date of publication and mention of the grant of the patent: **01.07.2026**
(21) Application number: 23776982.3
(22) Date of filing: 27.09.2023
(51) Int. Cl.: C07C 237/30

(54) **TERTIARY AMINE DERIVATIVES AND THEIR USES FOR TREATING A VIRAL INFECTION**
TERTIÄRE AMINDERIVATE UND IHRE VERWENDUNGEN ZUR BEHANDLUNG EINER VIRUSINFEKTION
DÉRIVÉS D'AMINES TERTIAIRES ET LEURS UTILISATIONS POUR LE TRAITEMENT D'UNE INFECTION VIRALE

(30) Priority: 27.09.2022 EP 22306428
(43) Date of publication of application: 06.08.2025
(73) Proprietor: ENYO Pharma, 69003 Lyon (FR)
(72) Inventor: DE CHASSEY, Benoît, 69004 LYON (FR); GUILLIER, Fabrice, 21000 DIJON (FR); MASSARDIER, Christine, 21000 DIJON (FR); MACHIN, Peter, deceased (FR); MELDRUM, Eric, 4125 Riehen (CH)
(74) Representative: Cabinet Becker et Associés
(86) International application number: PCT/EP2023/076639
(87) International publication number: WO 2024/068692

(56) References cited:
- WO-A1-2020/260526

## Description

### FIELD OF THE INVENTION

The present invention relates to the field of medicine, in particular tertiary amine derivatives and their uses for treating a viral infection, particularly a viral respiratory infection.

### BACKGROUND OF THE INVENTION

Viruses are small infectious agents that replicate only inside living cells of other organisms. They can infect all types of life forms, from animals and plants to microorganisms, including bacteria and archaea. Among them, more than 400 species of virus are known to be responsible of diseases in humans, many of them leading to serious pathologies and eventually death. In particular, HIV was classified at the sixth leading cause of death worldwide in 2012 with 1.5 millions of deaths per year (WHO, Fact sheet N°310, 2014). Seasonal influenza viruses are responsible of flu that affects approximately 20% of the world population and causes 250,000 to 500,000 deaths per year (WHO, Fact sheet N°211, 2014). Hepatitis B and C are responsible altogether for about 1.4 million of death each year and human Papillomaviruses are responsible of cervix cancer, the second most common women cancer worldwide, leading to 270,000 death in 2012 (WHO, Fact sheets, 2016).

A further particular serious virus is the human respiratory syncytial virus (HRSV) that causes respiratory infections, particularly during infancy and childhood. By the age of 1 year, 60-70% of children have been infected by HRSV. Complications associated with HRSV are for instance bronchiolitis, pneumonia, recurring respiratory infections, and otitis.

Because viruses use vital metabolic pathways within host cells to replicate, they are difficult to eliminate without using drugs that cause toxic effects to host cells in general. The most effective medical approaches to viral diseases are vaccinations to provide immunity to infection, and antiviral drugs that selectively interfere with viral replication. Vaccines are very effective on stable viruses for a preventive use. However, vaccines are of limited use in treating a patient who has already been infected. They are also difficult to successfully deploy against rapidly mutating viruses, and antiviral drugs may be particularly useful in these cases.

Antiviral drugs are a class of medication used specifically for treating viral infections. Antiviral drugs do not destroy their target pathogens, instead they inhibit their development. Antiviral drugs may target any stage of the viral life cycle: attachment to a host cell, release of viral genes and possibly enzymes into the host cell, replication of viral components using host-cell machinery, assembly of viral components into complete viral particles, and release of viral particles to infect new host cells. The most common antiviral drugs are nucleoside analogues that block viruses' replication. Most antiviral drugs are used for specific viral infections, while broad-spectrum antiviral drugs are effective against a wide range of viruses.

A commercially available antiviral drug to prevent HRSV is palivizumab, which is a monoclonal antibody. Palivizumab is administered by monthly injections just prior to the RSV season and this prevention is usually continued for a few months, generally for five months. However, the costs of palivizumab limits its use in many parts of the world. At this date, adrenaline, bronchodilators, steroids, antibiotics, ribavirin are currently used to treat RSV. However, they are limited to supportive measures and confer no real benefit for the subject.

Thus, there is nowadays a strong need for the development of new antiviral drugs, and in particular HRSV antiviral drugs. Also, the emergence of drug resistance poses a critical limitation on the application of antiviral drugs and have raised an urgent need for developing new anti-viral drugs against resistant forms.

In this context, tertiary amine derivatives have been provided and are disclosed in the PCT application WO 2020/260526. These derivatives exhibit an efficient antiviral effect against HRSV, which still remains to be improved.

### SUMMARY OF THE INVENTION

As illustrated by examples, the inventors have demonstrated the therapeutic interest of the compound of formula (I) according to the invention. Surprisingly, this specific compound exhibits a higher efficacy against respiratory syncytial virus (RSV) compared to compounds of WO 2020/260526, and positive control compounds Ribavirin and Presatovir.

The present invention thus provides a compound of formula (I): 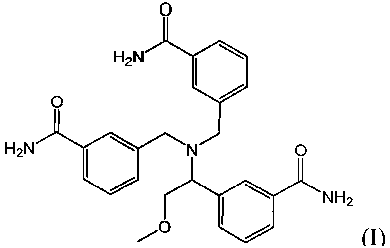 and the stereoisomers and the pharmaceutical salts thereof.

In a preferred embodiment, a compound of formula (I) is of formula (IA):

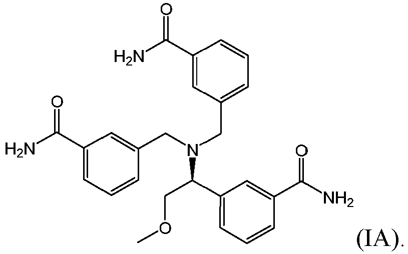

In a further preferred, a compound of formula (I) is of formula (IB):

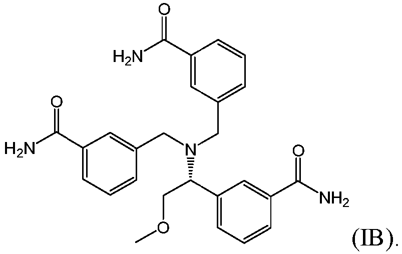

Another object of the invention is a compound of formula (I) as defined above for use as a medicine. A further object of the invention is a pharmaceutical composition comprising a compound of formula (I) as defined above, and an acceptable pharmaceutical excipient. In another further particular embodiment, the present invention relates to a compound or a pharmaceutical composition of the present invention for use for treating a viral infection. Preferably the viral infection is a viral respiratory infection. More preferably, the viral respiratory infection is caused by Human Respiratory Syncytial Virus.

### LEGENDS OF FIGURES

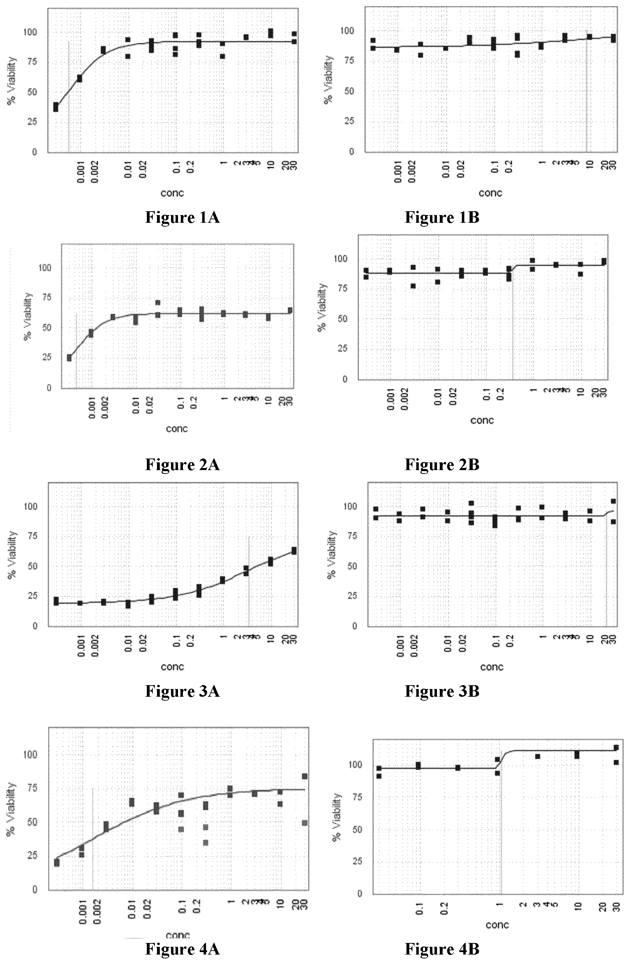

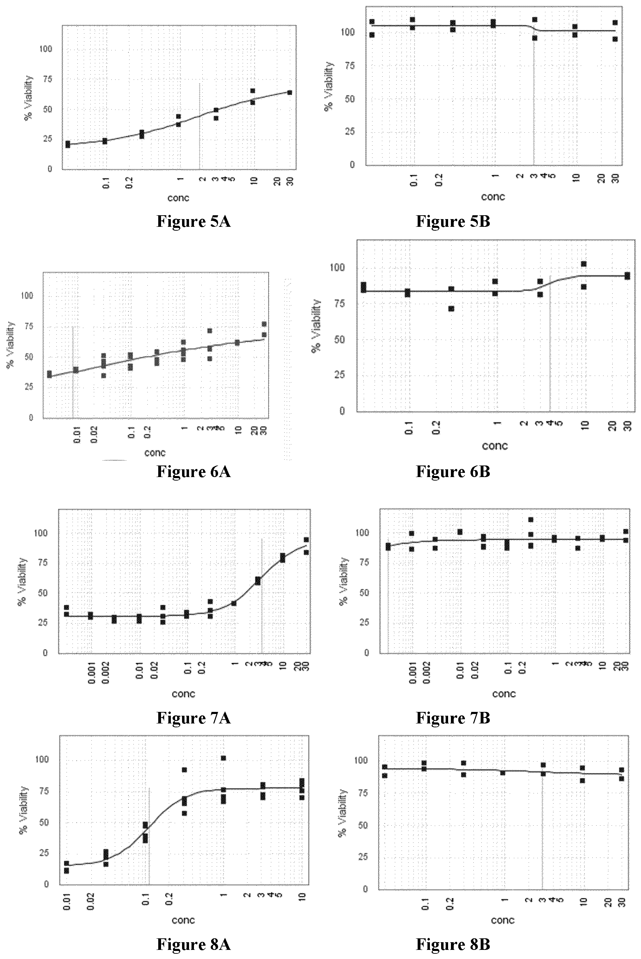

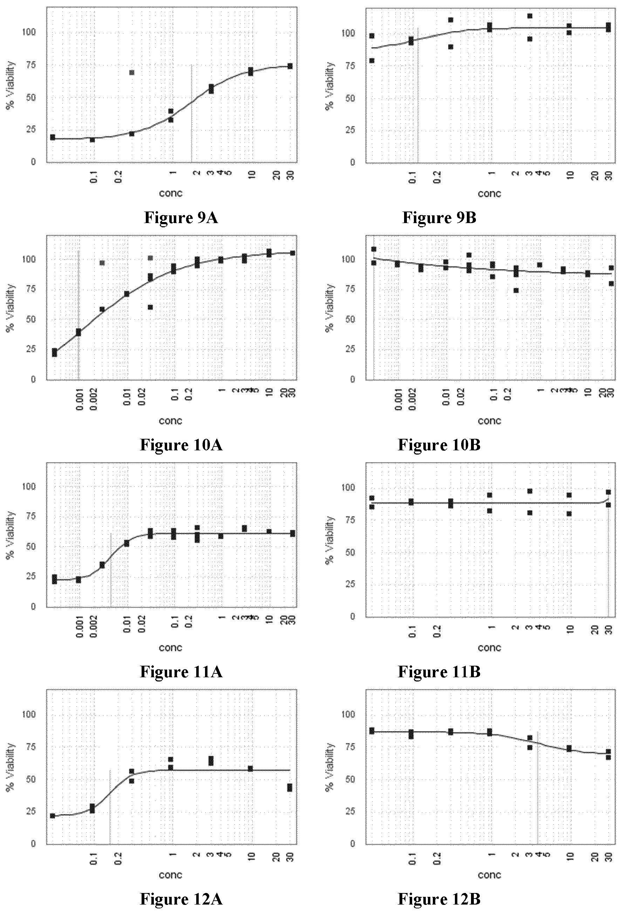

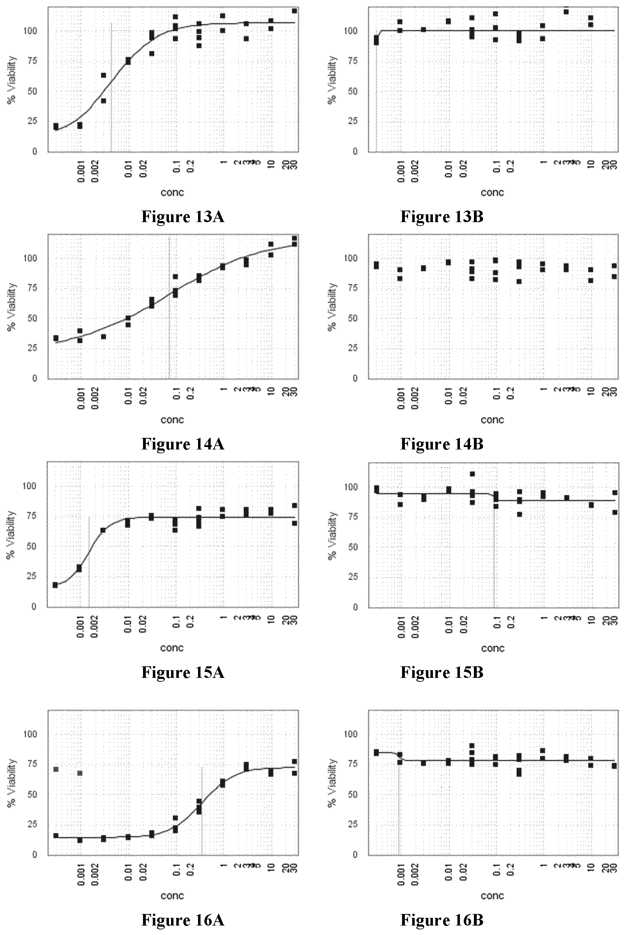

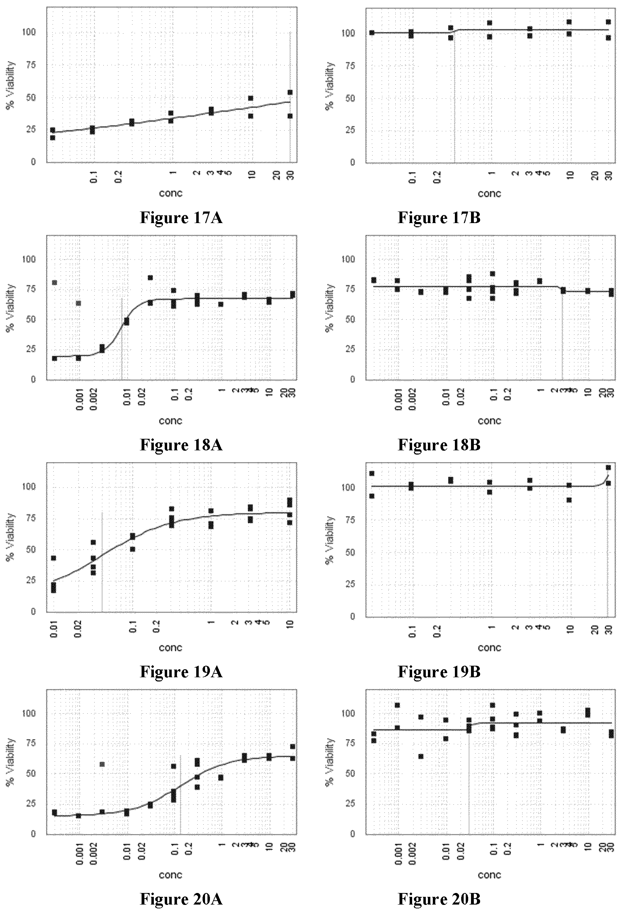

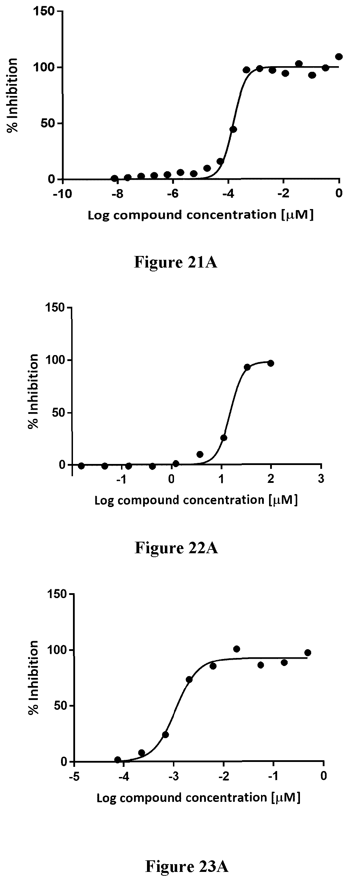

**Figures 1-23****:** % viability of cells infected by RSV (A) or cells not infected (B) after treating by comparative compounds (Compound #1, Figures 1A and 1B; Compound #2, Figures 2A and 2B; Compound #3, Figures 3A and 3B; Compound #4, Figures 4A and 4B; Compound #5, Figures 5A and 5B; Compound #6, Figures 6A and 6B; Compound #7, Figures 7A and 7B; Compound #8, Figures 8A and 8B; Compound #9, Figures 9A and 9B; Compound #10, Figures 10A and 10B; Compound #11, Figures 11A and 11B; Compound #12, Figures 12A and 12B; Compound #13, Figures 13A and 13B; Compound #14, Figures 14A and 14B; Compound #15, Figures 15A and 15B; Compound #16, Figures 16A and 16B; Compound #17, Figures 17A and 17B; Compound #18, Figures 18A and 18B, Compound #19, Figures 19A and 19B, Compound #20, Figures 20A and 20B), the compound of the invention (Compound #21, Figure 21A), and positive control compounds (Ribavirin, Figure 22A; Presatovir, Figure 23A).

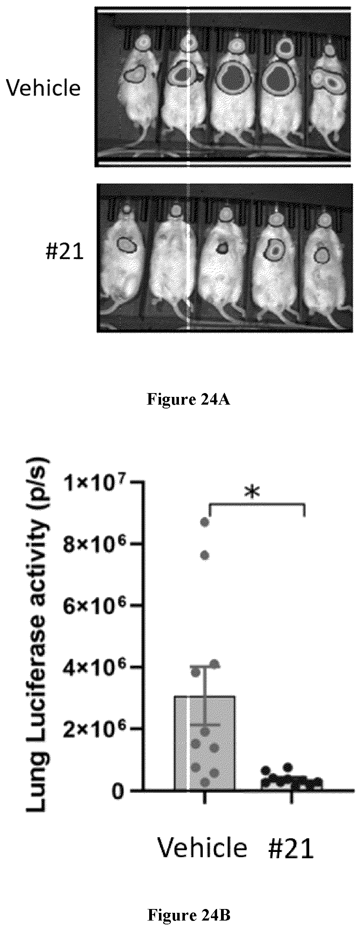

**Figure 24****:** In vivo evaluation of compound #21 anti-hRSV activity in a mouse model of infection. Figure 24A: Bioluminescence was measured by intranasal injection of 50 µl of D-luciferin (200 mM) and capture of photon emission from the chest at 5 days post infection using the IVIS system. Dorsal and ventral views of mice representative of the results are shown. Figure 24B: Comparative bioluminescence between compound #21 and mock-treated mice 5 days post infection. Each data point represents mean±s.e.m. Brown-Forsythe Test and Welch ANOVA followed by Dunnett's multiple comparison test; * p<0.05

### DETAILED DESCRIPTION OF THE INVENTION

### Definitions

The "stereoisomers" are isomeric compounds that have the same molecular formula and sequence of bonded atoms, but differ in the 3D-dimensional orientations of their atoms in space. The stereoisomers include enantiomers, diastereoisomers, Cis-trans and E-Z isomers, conformers, and anomers. In a preferred embodiment of the invention, the stereoisomers include diastereoisomers and enantiomers.

The "pharmaceutically salts" include inorganic as well as organic acids salts. Representative examples of suitable inorganic acids include hydrochloric, hydrobromic, hydroiodic, phosphoric, and the like. Representative examples of suitable organic acids include formic, acetic, trichloroacetic, trifluoroacetic, propionic, benzoic, cinnamic, citric, fumaric, maleic, methanesulfonic and the like. Further examples of pharmaceutically inorganic or organic acid addition salts include the pharmaceutically salts listed in J. Pharm. Sci. 1977, 66, 2, and in Handbook of Pharmaceutical Salts: Properties, Selection, and Use edited by P. Heinrich Stahl and Camille G. Wermuth 2002. In a preferred embodiment, the salt is selected from the group consisting of maleate, chlorhydrate, bromhydrate, and methanesulfonate. The "pharmaceutically salts" also include inorganic as well as organic base salts. Representative examples of suitable inorganic bases include sodium or potassium salt, an alkaline earth metal salt, such as a calcium or magnesium salt, or an ammonium salt. Representative examples of suitable salts with an organic base includes for instance a salt with methylamine, dimethylamine, trimethylamine, piperidine, morpholine or tris-(2-hydroxyethyl)amine. In a preferred embodiment, the salt is selected from the group consisting of sodium and potassium salt.

As used herein, the terms "treatment", "treat" or "treating" refer to any act intended to ameliorate the health status of patients such as therapy, prevention, prophylaxis and retardation of a disease, in particular a viral infection. In certain embodiments, such terms refer to the amelioration or eradication of the disease, or symptoms associated with it. In other embodiments, this term refers to minimizing the spread or worsening of the disease, resulting from the administration of one or more therapeutic agents to a subject with such a disease.

As used herein, the terms "subject", "individual" or "patient" are interchangeable and refer to an animal, preferably to a mammal, even more preferably to a human, including adult, child, newborn and human at the prenatal stage. However, the term "subject" can also refer to non-human animals, in particular mammals such as dogs, cats, horses, cows, pigs, sheep and non-human primates, among others.

The terms "quantity," "amount," and "dose" are used interchangeably herein and may refer to an absolute quantification of a molecule.

As used herein, the terms "active principle", "active ingredient" and "active pharmaceutical ingredient" are equivalent and refers to a component of a pharmaceutical composition having a therapeutic effect.

As used herein, the term "therapeutic effect" refers to an effect induced by an active ingredient, or a pharmaceutical composition according to the invention, capable to prevent or to delay the appearance or development of a disease or disorder, or to cure or to attenuate the effects of a disease or disorder.

As used herein, the term "effective amount" refers to a quantity of an active ingredient or of a pharmaceutical composition which prevents, removes or reduces the deleterious effects of the disease, particularly infectious disease. It is obvious that the quantity to be administered can be adapted by the man skilled in the art according to the subject to be treated, to the nature of the disease, etc. In particular, doses and regimen of administration may be function of the nature, of the stage and of the severity of the disease to be treated, as well as of the weight, the age and the global health of the subject to be treated, as well as of the judgment of the doctor.

As used herein, the term "pharmaceutically acceptable excipient" refers to any ingredient except active ingredients that is present in a pharmaceutical composition. Its addition may be aimed to confer a particular consistency or other physical or gustative properties to the final product. A pharmaceutically acceptable excipient must be devoid of any interaction, in particular chemical, with the active ingredients.

According to the invention, the compound has the following formula (I):

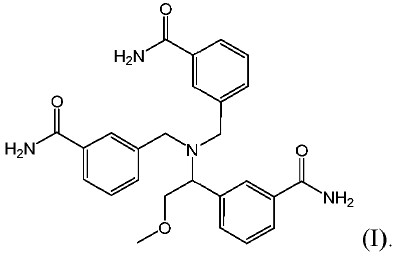

In a particular embodiment, the compound of formula (I) further includes stereoisomers, such as enantiomers and diastereoisomers.

In a preferred embodiment, the compound of formula (I) is of formula (IA):

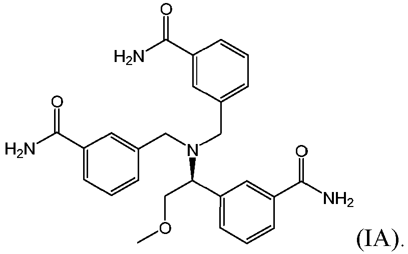

The compound of formula (IA) has the following chemical name: 3-[[[(1S)-1-(3-carbamoylphenyl)-2-methoxy-ethyl]-[(3-carbamoylphenyl) methyl]amino]methyl]benzamide. The compound of formula (IA) is also called herein compound #21 or compound 7.

In a further preferred, a compound of formula (I) is of formula (IB):

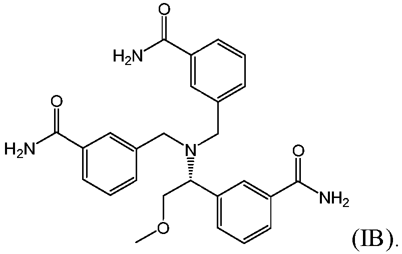

The compound of formula (IB) has the following chemical name: 3-[[[(1R)-1-(3-carbamoylphenyl)-2-methoxy-ethyl]-[(3-carbamoylphenyl)methyl]amino]methyl]benzamide.

The compound of formula (I) according to the present invention can be prepared according to any chemical routes known from a skilled person, such the synthetic route presented in the examples. It is thus understood that one skilled in the art of organic chemistry can easily synthesize the compound of formula (I) using appropriate starting materials, conventional chemicals reactions, standard and literatures procedures, and experimental conditions to synthesize the compounds of formula (I).

As illustrated by examples, the inventors have demonstrated the therapeutic interest of the new compound of the invention. Indeed, the inventors show that the compound according to the present invention has an improved antiviral activity, especially against the Respiratory Syncytial Virus. Therefore, the compounds of the present invention are useful as a drug, especially as antiviral agent.

Accordingly, the present invention relates to a compound as defined herein, for use as a drug or a medicine. The present invention further relates to a pharmaceutical or veterinary composition comprising a compound according to the invention. Preferably, the pharmaceutical composition further comprises a pharmaceutically or veterinary acceptable carrier or excipient. The present invention relates to the use of a compound according to the invention as a drug or a medicine. The invention further relates to the use in a method for treating a disease in a subject, wherein a therapeutically effective amount of a compound according to the invention, is administered to said subject in need thereof. The invention also relates to the use of a compound according to the invention, for the manufacture of a medicine. The invention also relates to a pharmaceutical composition comprising a compound according to the invention for use as a drug.

The present invention relates to a compound according to the invention for use for treating a viral infection. It further relates to the use of a compound according to the invention, for the manufacture of a medicine for treating a viral infection. It also relates to a pharmaceutical composition comprising a compound according to the invention for use for treating a viral infection. Finally, it relates to a method for treating a viral infection in a subject in need thereof, wherein a therapeutically effective amount of a compound according to the invention, is administered to said subject in need thereof.

The present invention relates to the use of a compound according to the invention as an antiviral agent. The present invention also relates to the use of a compound of the present invention as a research tool, especially for studying viral infections. It further relates to the use in a method for blocking viral infection in a cell, a tissue or a subject.

The viral agent can be a DNA virus or a RNA virus.

The viral agent can be selected from the group consisting of Alphaviridae, Flaviviridae, Hepadnaviridae, Herpesviridae, Orthomyxoviridae, Papovaviridae, Paramyxoviridae, Picornaviridae, Polyomaviridae, Reoviridae, Retroviridae, Rhabdoviridae, and Tobamoviruses.

In one embodiment, the Alphaviridae is selected from the group consisting of Barmah Forest virus, Middelburg virus, Ndumu virus, Bebaru virus, Chikungunya virus, Mayaro virus, O'nyong'nyong virus, Ross River virus, Semliki Forest virus, Sindbis virus, Una virus, Eastern equine encephalitis virus, Tonate virus, Venezuelan equine encephalitis virus, Cabassou virus, Everglades virus, Mosso das Pedras virus, Mucambo virus, Parmana virus, Pixuna virus, Rio Negro virus, Trocara virus, Aura virus, Babanki virus, Kyzylagach virus, Ockelbo virus, Whataroa virus, Sleeping disease virus, Samon pancreatic disease virus, Southern elephant seal virus, and Western equine encephalitis virus; preferably selected from the group consisting of Barmah Forest virus, Chikungunya virus, Mayaro virus, O'nyong'nyong virus, Ross River virus, Semliki Forest virus, Sindbis virus, Una virus, Eastern equine encephalitis virus, Tonate virus, Venezuelan equine encephalitis virus and Western equine encephalitis virus.

In one embodiment, the Flaviviridae is selected from the group consisting of dengue virus, Hepatitis C virus, Japanese encephalitis virus, West Nile virus, yellow fever virus, Zika virus, Tick-borne encephalitis virus, Kyasanur forest disease virus, Murray Valley encephalitis virus, and Saint Louis encephalitis virus.

In one embodiment, the Hepadnaviridae is selected from the group consisting of Hepatitis B virus.

In one embodiment, the Herpesviridae is selected from the group consisting of Herpes Simplex virus 1 (HSV-1), Herpes Simplex virus 2 (HSV-2), Varicella zoster virus (VZV), Epstein-Barr virus (EBV), Cytomegalovirus (CMV), Roseolovirus (HHV-6A and 6B), HHV-7 and Kaposi's sarcoma-associated herpesvirus (KSHV).

In one embodiment, the Orthomyxoviridae is selected from the group consisting of Influenza virus A, Influenza virus B, Influenza virus C, Isavirus, Thogotovirus and Quaranjavirus, preferably selected from the group consisting of Influenza virus A and Influenza virus B. In one embodiment, the Influenza virus A is selected from the subtypes consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7.

In one embodiment, the Papovaviridae is selected from the group consisting of Papillomavirus (HPC) and Polyomavirus, especially Simian virus 40, Merkel cell polyomavirus, Trichodysplasia spinulosa polyomavirus, BK polyomavirus, JC polyomavirus and Human polyomavirus 7.

In one embodiment, the Picornaviridae is selected from the group consisting of Aphthovirus, Aquamavirus, Avihepatovirus, Cardiovirus, Cosavirus, Dicipivirus, Enterovirus, Erbovirus, Hepatovirus, Kobuvirus, Megrivirus, Parechovirus, Piscevirus, Rhinovirus, Salivirus, Sapelovirus, Senecavirus, Techovirus, and Tremovirus. In a particular embodiment, the Picornaviridae is a Rhinovirus, for instance a Rhinovirus A, Rhinovirus B or Rhinovirus C.

In one embodiment, the Retroviridae is selected from the group consisting of Alpharetrovirus; especially Avian leukosis virus and Rous sarcoma virus; Betaretrovirus, especially Mouse mammary tumour virus; Gammaretrovirus, especially Murine leukemia virus and Feline leukemia virus; Deltaretrovirus, especially Bovine leukemia virus and Human T-lymphotropic virus; Epsilonretrovirus, especially Walleye dermal sarcoma virus; Lentivirus, especially Human immunodeficiency virus 1 and Simian, Feline immunodeficiency viruses; Spumavirus, especially Simian foamy virus.

In one embodiment, the Rhabdoviridae is selected from the group consisting of vesiculovirus, especially vesicular stomatitis virus, lyssavirus, rabies virus, Ephemerovirus, novirhabdovirus, cytorhabdovirus and nucleorhabdovirus.

In one embodiment, the viral agent according to the invention is selected from the group consisting in Herpesviridae such as Varicella zoster virus (VZV), Epstein-Barr (EB) virus, Herpes simplex virus of type 1 (HSV-1), Kaposis sarcoma herpesvirus (KSHV), murine γ-HV68 virus (γ-MHV68), or human cytomegalovirus (HCMV); Hepadnaviridae such as Hepatitis virus B (HBV); Papovaviridae such as Human papillomavirus type 16 (HPV16); Parvoviridae such as Human parvovirus B19; Polyomaviridae such as Simian virus 40; Retroviridae such has Human immunodeficiency virus 1 (HIV-1), or Simian immunodeficiency virus type 1 (SIV 1); Orthomyxoviridae such as Influenza A virus; Flaviviridae such as Dengue virus, or Hepatitis C virus; Picornaviridae such as Poliovirus, Coxsakievirus B3 (CVB3), or Coxsakievirus B4 (CVB4); Reoviridae such as Rotavirus; Alphaviridae such as Sindbis virus; Tobamoviruses such as Tabacco mosaic virus; Rhabdoviridae such as vesicular stomatitis virus. More preferably, the viral agent according to the invention is an influenza virus. Still preferably, the viral agent according to the invention is an influenza virus A or B, even more preferably an influenza virus A.

In a preferred embodiment, the virus is a Paramyxoviridae. The Paramyxoviridae can be selected from the group consisting of Rubulavirus, Morbillivirus, Pneumovirus, Metapneumovirus, Avulavirus, Ferlavirus, Henipavirus, and Respirovirus. In a particular embodiment, the Paramyxoviridae is the mumps virus, measles virus, human parainfluenza viruses (HPIV), especially HPIV-1, HPIV-2, HPIV-3 or HPIV-4, respiratory syncytial virus (RSV), in particular Human respiratory syncytial virus (HRSV), canine distemper virus, phocine distemper virus, cetacean morbillivirus, Newcastle disease virus, rinderpest virus, Hendra birus and Nipah virus. In a preferred embodiment, the viral agent is respiratory syncytial virus (RSV), in particular Human respiratory syncytial virus (HRSV). In a preferred aspect, the virus is respiratory syncytial virus (RSV), in particular Human respiratory syncytial virus (HRSV) and the subject suffers from a bronchitis, a bronchiolitis or a pneumonia.

In one embodiment, the compound of the invention can be used in combination with another antiviral drug, for instance and non-exhaustively, an agent selected from the group consisting of neuraminidase inhibitors, M2 inhibitors, RNA polymerase inhibitors, interferons (immune system modulators interferon alpha-2a and PEGylated interferon alpha-2a (Pegasys) and interferon alpha-2b (ViraferonPeg ou Introna)), antiviral vaccine, antigenic polypeptides or neutralizing antibodies directed to a viral antigenic polypeptide. More particularly, in the case of RSV infection, the compound according to the invention could be combined with palivizumab. In addition or alternatively, it may also be combined with adrenaline, bronchodilatators, steroids, antibiotics and/or an antiviral drug, in particular a nucleoside analog such as ribavirin.

The compound according to the invention or the pharmaceutical composition according to the invention may be administered by any conventional route of administration. In particular, the compound or the pharmaceutical composition of the invention can be administered by a topical, enteral, oral, parenteral, intranasal, intravenous, intra-arterial, intramuscular, intratumoral, subcutaneous or intraocular administration and the like.

In particular, the compound according to the invention or the pharmaceutical composition according to the invention can be formulated for a topical, enteral, oral, parenteral, intranasal, intravenous, intra-arterial, intramuscular, intratumoral, subcutaneous or intraocular administration and the like.

Preferably, the compound according to the invention or the pharmaceutical composition according to the invention is administered by enteral or parenteral route of administration. When administered parenterally, the compound according to the invention or the pharmaceutical composition according to the invention is preferably administered by intravenous route of administration. When administered enterally, the compound according to the invention or the pharmaceutical composition according to the invention is preferably administered by oral route of administration.

The pharmaceutical composition comprising the molecule is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

For oral administration, the composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

Preferably, the treatment with the compound according to the invention or the pharmaceutical composition according to the invention starts no longer than a month, preferably no longer than a week, after the diagnosis of the disease. In a most preferred embodiment, the treatment starts the day of the diagnosis.

The compound according to the invention or the pharmaceutical composition according to the invention may be administered as a single dose or in multiple doses.

Preferably, the treatment is administered regularly, preferably between every day and every month, more preferably between every day and every two weeks, more preferably between every day and every week, even more preferably the treatment is administered every day. In a particular embodiment, the treatment is administered several times a day, preferably 2 or 3 times a day, even more preferably 3 times a day.

The duration of treatment with the compound according to the invention or the pharmaceutical composition according to the invention is preferably comprised between 1 day and 20 weeks, more preferably between 1 day and 10 weeks, still more preferably between 1 day and 4 weeks, even more preferably between 1 day and 2 weeks. In a particular embodiment, the duration of the treatment is of about 1 week. Alternatively, the treatment may last as long as the disease persists.

The amount of compound according to the invention or of pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

In a preferred embodiment, the total compound dose for each administration of the compound according to the invention or of the pharmaceutical composition according to the invention is comprised between 0.00001 and 1 g, preferably between 0.01 and 10 mg.

The form of the pharmaceutical compositions, the route of administration and the dose of administration of the compound according to the invention, or the pharmaceutical composition according to the invention can be adjusted by the man skilled in the art according to the type and severity of the disease, and to the patient, in particular its age, weight, sex, and general physical condition.

The present invention also relates to the combined use of a compound of the present invention with at least another active ingredient, preferably an antiviral agent, for the treatment of a viral infection, preferably a viral respiratory infection.

The present invention also relates to a product comprising a compound of the present invention, and another active ingredient, as a combined preparation for simultaneous, separate or sequential use, in particular for use for the treatment of a viral disease or a viral infection. Preferably, the other active ingredient is an antiviral agent.

Further aspects and advantages of the present invention will be described in the following examples, which should be regarded as illustrative and not limiting.

### EXAMPLES

### EXAMPLE A - CHEMISTRY

### Compound #21: 3-[[[(1S)-1-(3-carbamoylphenyl)-2-methoxy-ethyl]-[(3-carbamoylphenyl) methyl]amino]methyl]benzamide (also referenced compound 7 in the scheme below).

### Abbreviations:

AcOH = Acetic acid; ACN = Acetonitrile; DCM = Dichloromethane; d = Doublet; dd = Doublet of doublets; DMF = N,N-Dimethylformamide; DMSO = Dimethylsulfoxide; eq = Equivalent(s); EtOAc = Ethyl acetate; g = Gram(s); h = Hour(s); MHz = MegaHertz; HCOOH = formic acid; K₂CO₃ = Potassium carbonate; LC/MS = Liquid chromatography/mass spectrometry; LiOH = Lithium hydroxide; m = Multiplet; M = Molar; min = Minute; MeI = MethylIodide; mg = Milligram; MgSO₄ = Magnesium sulfate; mL = Millilitre; mmol = Millimole; mol/L = Moles/Litre; NaH = Sodium hydride; NH₄Cl = Ammonium chloride; NMR = Nuclear magnetic resonance; ppm = Parts per million; q = Quadruplet ; quant = Quantitative ; quint = Quintuplet ; Rt = Retention time ; rt = Room temperature; s = singlet; t = triplet.

### Analytical Methods:

Analytical data is included within the procedures below, in the illustrations of the general procedures, or in the tables of examples. Unless otherwise stated, all ¹H NMR data were collected on a Bruker AVIII 400MHz equipped with 5mm BB(O)F GRADZ probe and chemical shifts are quoted in parts per million (ppm). LC/MS was performed UPLC Acquity Waters a PDA Acquity detector and a SQ Acquity mass spectrometer.

### Synthesis Scheme:

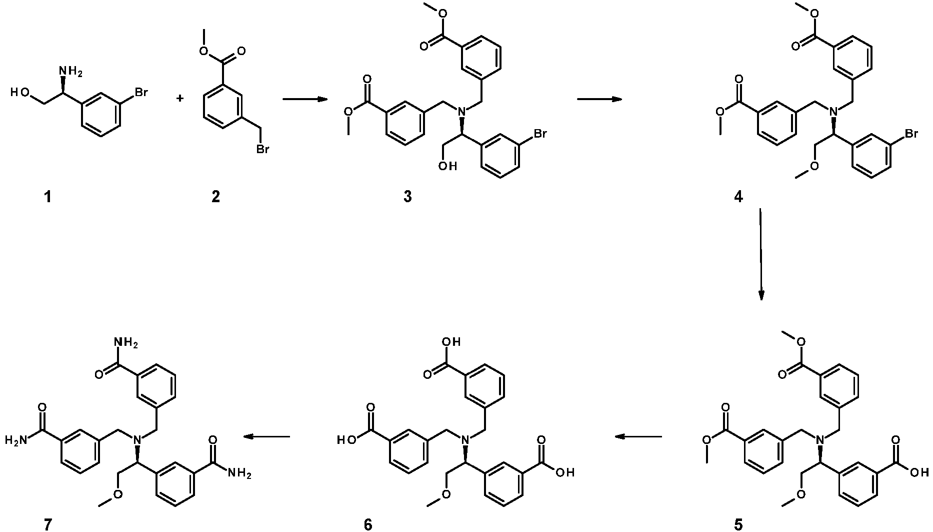

### Synthesis Protocols:

Reagents and solvents obtained from commercial suppliers are used without further purification.

### Compound 3: methyl 3-[[[(1S)-1-(3-bromophenyl)-2-hydroxy-ethyl]-[(3-methoxycarbonylphenyl) methyl]amino]methyl]benzoate.

In a 100ml round-bottomed flask, with condenser, under N₂ atm, (2s)-2-amino-2-(3-bromophenyl)ethan-1-ol 1 (1.00 g; 4.63 mmol; 1 eq.) was dissolved in 20ml acetonitrile. Potassium carbonate (1.92g; 13.88 mmol; 3 eq.) and methyl-3-bromomethyl benzoate 2 (2.33g; 10.18 mmol; 2.2 eq.) were added. the reaction mixture was stirred at 70°C overnight. The reaction mixture was then concentrated under reduced pressure. The residue was diluted in water and EtOAc. The Layers were separated and the organic Layer was washed with brine, dried over MgSO₄, filtered and concentrated under reduced pressure to provide the crude product. This product was purified by flash chromatography on Si cartridge SNAP Ultra 100g with Cyclohexane/EtOAc gradient from 15% to 60% at 60mL/min to provide the expected product **3** (2.04 g; 86 %) as an amorphous beige solid. LCMS ¹H NMR (300 MHz, DMSO-*d*₆) δ ppm. ¹H NMR (300 MHz, DMSO-d₆) δ ppm 3.47 (d, *J*=14.20 Hz, 2 H) 3.66 - 3.76 (m, 1 H) 3.78 - 3.88 (m, 8 H) 3.90 - 4.02 (m, 2 H) 4.72 (t, *J*=5.12 Hz, 1 H) 7.31 - 7.39 (m, 2 H) 7.41 - 7.52 (m, 3 H) 7.53 - 7.64 (m, 3 H) 7.80 (dt, *J*=7.84, 1.45 Hz, 2 H) 7.92 (t, *J*=1.49 Hz, 2 H).

### Compound 4: methyl 3-[[[(1S)-1-(3-bromophenyl)-2-methoxy-ethyl]-[(3-methoxycarbonyl-phenyl)methyl]amino]methyl]benzoate.

In a 50mL round-bottomed flask, under Ar atm, methyl 3-[[[(1S)-1-(3-bromophenyl)-2-hydroxy-ethyl]-[(3-methoxycarbonylphenyl)methyl]amino]methyl]benzoate **3** (811 mg; 1.58 mmol; 1 eq.) was dissolved in 24 mL of DMF and cooled at 0°C. NaH (60%) (108 mg; 2.69 mmol; 1.7 eq.) was added at 0°C. The reaction mixture was stirred at 0°C for 30min then MeI (167 µL; 2.69 mmol; 1.7 eq.) was added. The reaction mixture was further stirred at rt overnight. The reaction mixture was washed with Na₂S₂O₃ and extracted with EtOAc. The organic layer was washed with water then brine. The organic layer was dried over MgSO₄, filtered and concentrated under reduced pressure to provide the crude product. This product was purified by flash chromatography on Si cartridge SNAP Ultra 50g with Cyclohexane/EtOAc gradient from 5% to 40% at 50mL/min to provide the expected product **4** (375 mg; 45 %) as a yellow solid. ¹H NMR (300 MHz, DMSO-d₆) δ ppm 3.25 (s, 3 H) 3.48 (d, *J*=14.20 Hz, 2 H) 3.77 - 3.84 (m, 2 H) 3.78-3.85 (m, 8 H) 3.93 (m, 1 H) 7.35 (d, *J*=5.12 Hz, 2 H) 7.39 - 7.47 (m, 2 H) 7.47 - 7.61 (m, 4 H) 7.79 (dt, *J*=7.76, 1.40 Hz, 2 H) 7.93 (s, 2 H).

### Compound 5: 3-[(1S)- 1-[bis[(3-methoxycarbonylphenyl)methyl]amino]-2-methoxy-ethyl] benzoic acid.

Methyl-3-[[[(1S)-1-(3-bromophenyl)-2-methoxy-ethyl]-[(3-methoxycarbonylphenyl)methyl]amino] methyl]benzoate **4** (370 mg; 0.64 mmol; 1 eq.) was dissolved in DME (4.05 mL) in a 2mL microwave vial. Palladium acetate (14.36 mg; 0.06 mmol; 0.1 eq.), tri-tert-butylphosphonium tetrafluoroborate (18.5 mg; 0.06 mmol; 0.1 eq.), sodium carbonate (102 mg; 0.96 mmol; 1.5 eq.) and water (1.35 mL) were added. The reaction mixture was stirred at rt for 5min then Molybdenum hexacarbonyl (253mg; 0.96 mmol; 1.5 eq.) was added. The reaction mixture was irradiated under microwave at 140°C for 1h. The reaction mixture was washed with water and extracted with EtOAc (x2). The organic layer was dried over MgSO₄, filtered and concentrated under reduced pressure to provide the crude product. This product was purified by flash chromatography on Si cartridge SNAP 25g with Cyclohexane/EtOAc gradient from 15% to 65% at 40mL/min to provide the expected product **5** (197 mg; 63 %) as a white foam. ¹H NMR (300 MHz, DMSO-d₆) δ ppm 3.26 (s, 3 H) 3.48 (d, *J*=14.20 Hz, 2 H) 3.80 - 3.88 (m, 2 H) 3.85 (s, 6 H) 3.89 - 3.98 (m, 3 H) 7.37 - 7.49 (m, 2 H) 7.49 - 7.71 (m, 4 H) 7.73 - 7.84 (m, 2 H) 7.85 - 8.01 (m, 4 H) 12.88 - 13.04 (m, 1 H).

### Compound 6: 3-[[[(1S)-1-(3-carboxyphenyl)-2-methoxy-ethyl]-[(3-carboxyphenyl)methyl] amino]methyl]benzoic acid.

LiOH (29 mg; 1.20 mmol; 3 eq.) was added to a solution of 3-[(1S)- 1-[bis[(3-methoxycarbonylphenyl)methyl]amino]-2-methoxy-ethyl] benzoic acid **5** (197 mg; 0.40 mmol; 1 eq.) solubilized in a mixture of 4.7mL THF and 1.6mL water. The reaction was stirred at rt overnight. THF was evaporated and the residue was diluted with water. The aqueous phase was acified to pH 3 with HCl 1N and the crude product was extracted with DCM to provide the expected product 6 (185 mg; 99 %) as a pale yellow oil with a good enough purity. ¹H NMR (300 MHz, DMSO-*d*₆) δ ppm 3.24 (s, 3 H) 3.43 (br d, *J*=14.04 Hz, 2 H) 3.83 (d, *J*=14.20 Hz, 2 H) 3.88 - 3.98 (m, 3 H) 7.40 - 7.48 (m, 2 H) 7.48 - 7.56 (m, 1 H) 7.56 - 7.65 (m, 3 H) 7.80 (dt, *J*=7.76, 1.40 Hz, 2 H) 7.85 - 7.93 (m, 4 H) 12.54 - 13.29 (bs, 3 H).

### Compound 7 or #21: 3-[[[(1S)-1-(3-carbamoylphenyl)-2-methoxy-ethyl]-[(3-carbamoylphenyl) methyl]amino]methyl]benzamide.

To a solution of 3-[[[(1S)-1-(3-carboxyphenyl)-2-methoxy-ethyl]-[(3-carboxyphenyl)methyl] amino]methyl]benzoic acid **6** (165 mg; 0.36 mmol; 1 eq.) in 12mL DCM were added, 1-(3-dimethylaminopropyl)-3-ethylcarbodiimide hydrochloride (246 mg; 1.28 mmol; 3.6 eq.), 1-hydroxy-7-azabenzotriazole (174 mg; 1.28 mmol; 3.6 eq.), triethylamine (297 µL; 2.14 mmol; 6 eq.) and NH₄Cl (114 mg; 2.14 mmol; 6 eq.). The reaction was stirred at rt for overnight. The reaction was diluted with water and extracted with EtOAc (3x). the combined organic layers were dried over MgSO₄, filtered and concentrated to dryness to give 150 mg of crude product as a white powder. This product was purified by flash chromatography on Si cartridge SNAP 10g with DCM to DCM/MeOH (9/1) + NH₄OH 1% gradient from 0% to 30% at 15mL/min to provide the expected final product 7 3-[[[(1S)-1-(3-carbamoylphenyl)-2-methoxy-ethyl]-[(3-carbamoylphenyl)methyl] amino]methyl]benzamide (37 mg; 23 %)) as a white amorphous powder. ¹H NMR (400 MHz, DMSO-*d*₆) δ ppm 3.22 (s, 3 H) 3.36 (s, 2 H) 3.80 (d, *J*=14.20 Hz, 2 H) 3.86 - 3.96 (m, 3 H) 7.32 - 7.44 (m, 5 H) 7.47 (d, *J*=4.84 Hz, 2 H) 7.55 (d, *J*=7.70 Hz, 2 H) 7.73 (d, *J*=7.70 Hz, 2 H) 7.78 - 7.87 (m, 4 H) 7.91 (s, 2 H) 8.00 (s, 1 H). LC/MS analysis condition: Column: ACQUITY UPLC BEH C18, 50x2.1 mm, 1.7 µm, Temperature 45°C, Mobile phase: ACN (0.1% AcOH) in water (0.1% AcOH), from 5% to 95% within 2.50 min; Flow rate: 0.8 ml/min; Wavelength: 210-260 nm DAD. Rₜ = 1.01 min; MS *m*/*z:* 460 [M+H]⁺.

### Comparative compounds

Comparatives compounds #1-#20 have been prepared according to the procedures disclosed by WO 2020/260526. The chemical structure of comparative compounds #1-20 is provided below: 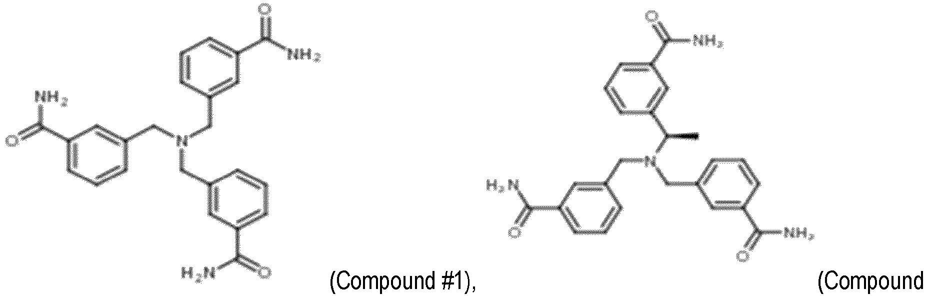 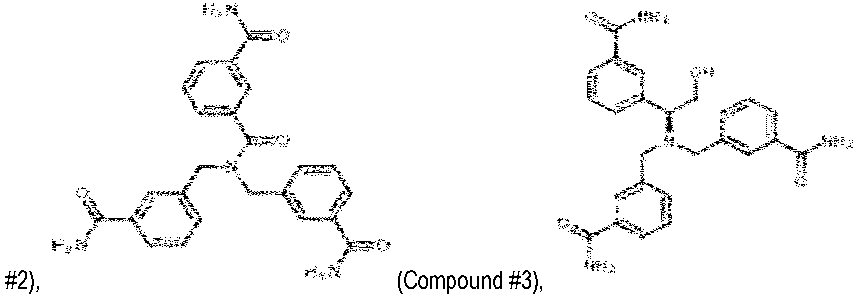 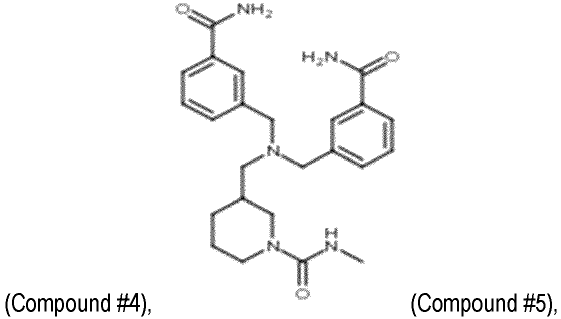 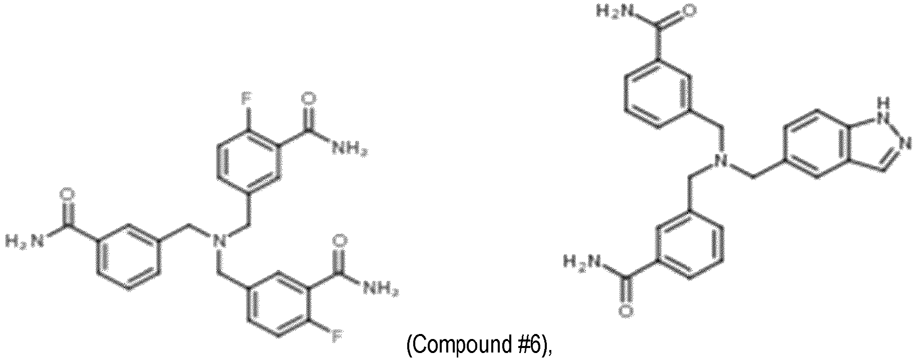 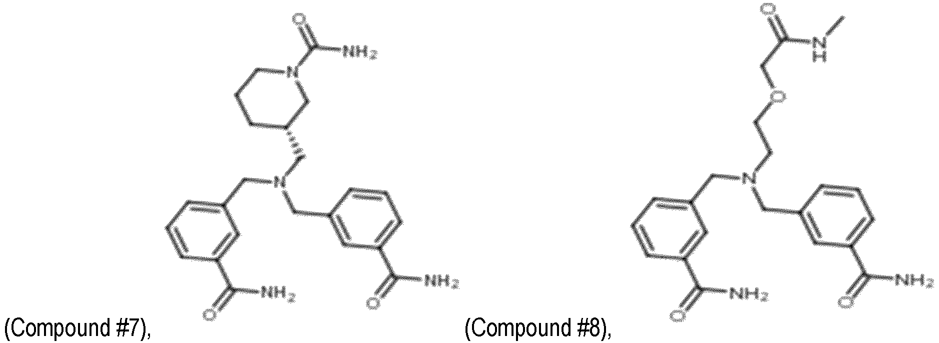 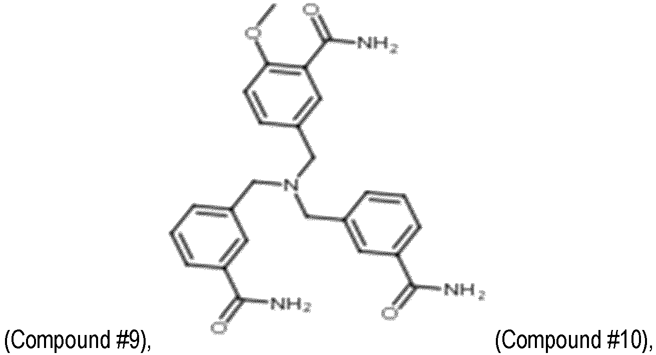 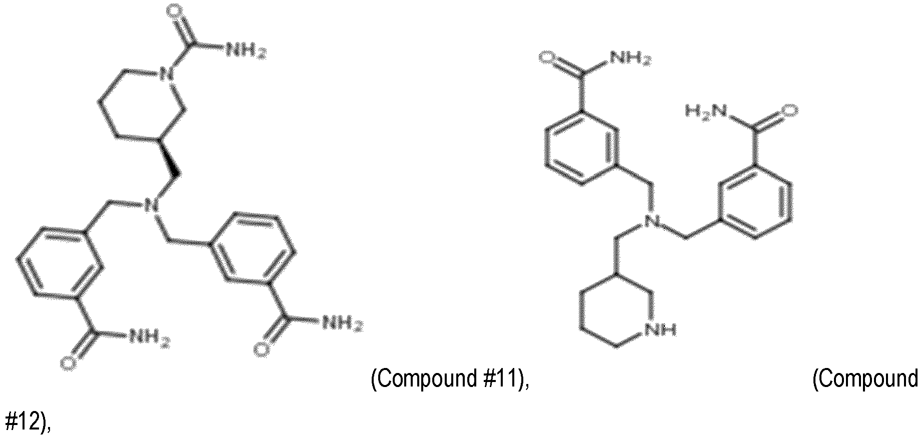 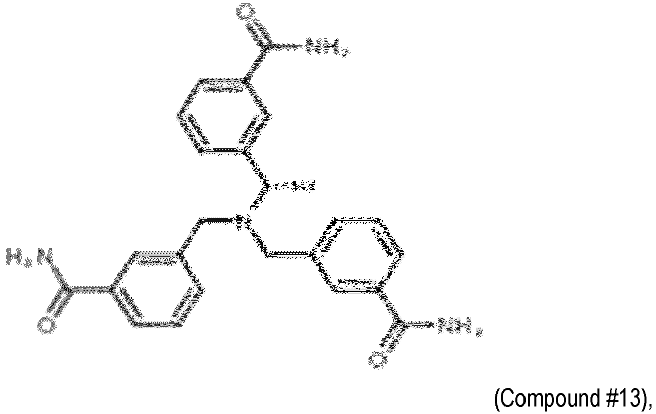 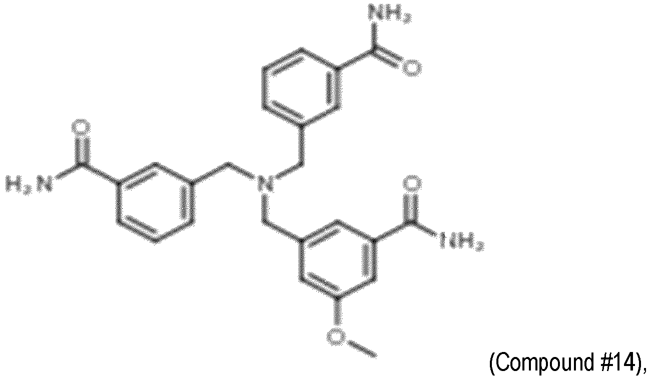 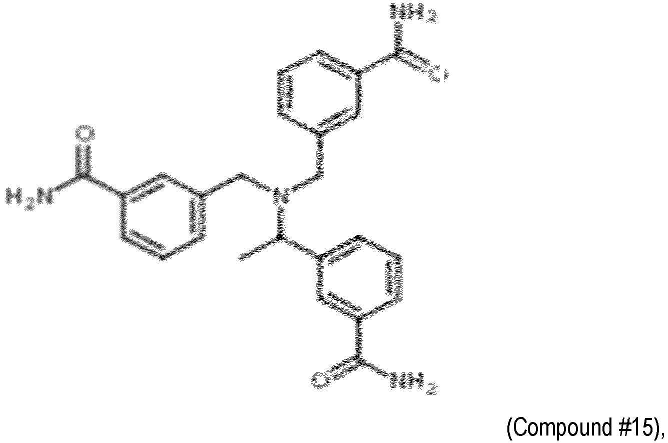 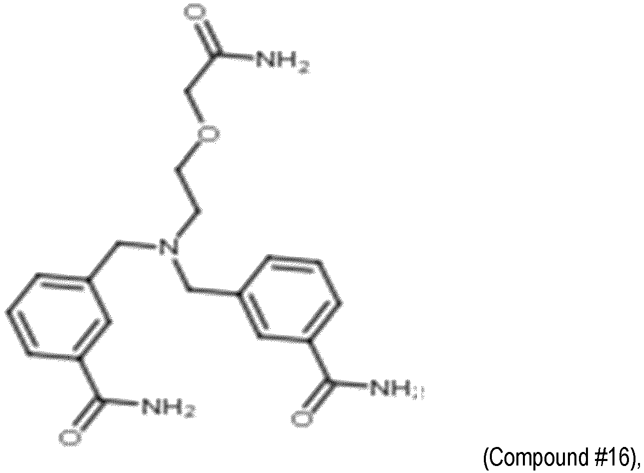 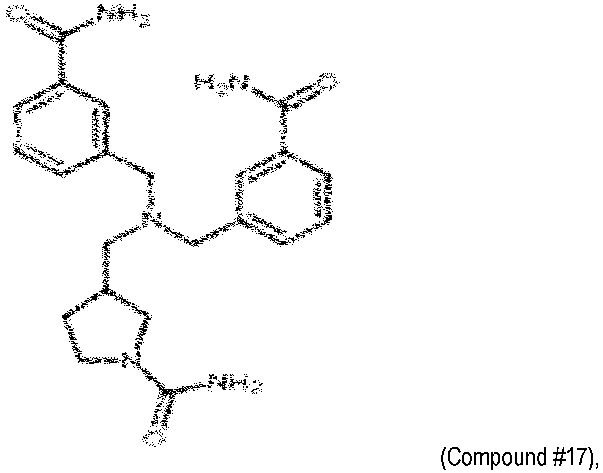 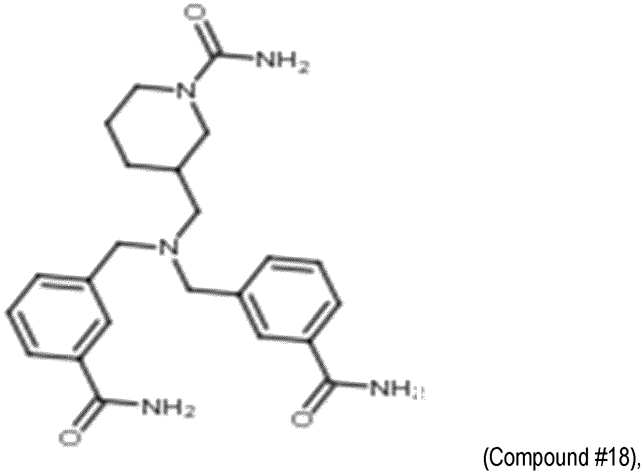 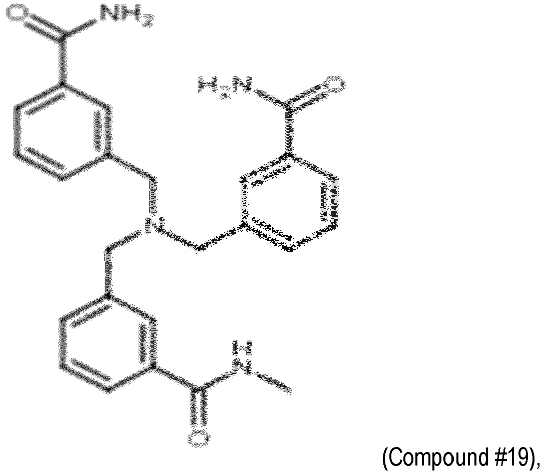 and

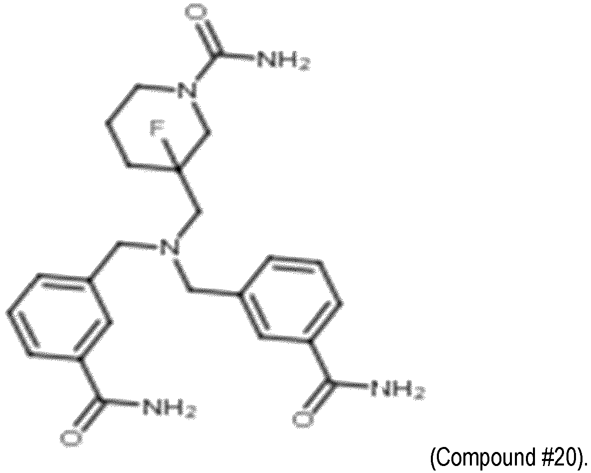

### EXAMPLE B - BIOLOGY

### Antiviral effect

### Material & methods

| **Reagent** | **Supplier** | **Code** | **Storage conditions** |
|---|---|---|---|
| HEp-2 cells | ATCC | CCl-23 | LN |
| Foetal bovine serum | Sigma | F4135 | -20°C |
| Eagle's Minimum Essential Medium (EMEM) | Sigma | 30-2003 | 4°C |
| 2x Eagle's Minimum Essential Medium (EMEM) | Lonza | LZBE12-668F | 4°C |
| L-glutamine | Sigma | G7513 | -20°C |
| Non-essential Amino Acid Solution (NEAA) | Sigma | M7145-100ML | 4°C |
| PBS without Ca & Mg | Sigma | D8537 | RT |
| Trypsin | Sigma | T4674 | 4°C |
| DMSO | Sigma | 276855 | RT |
| Crystal Violet | Fisher | 10626621 | RT |
| Penicillin-Streptomycin (Pen-Strep) | Sigma Aldrich | P4333 | -20°C |
| Trypan Blue | Sigma Aldrich | T8154 | RT |
| Hepes | Sigma | H0887-100ML | RT |
| Formaldehyde | Sigma | 252549-100ML | RT |
| Ribavirin | FluoroChem | 079125 | 4°C |
| Presatovir | MedChem | HY-16727 | -20°C |
| CellTiter-Glo^{®} | Promega | G7570 | -20°C |
| Red Fluorescent Protein (RFP) - Respiratory Syncytial Virus (RSV) | ViraTree | R131 | -80°C |
| 384-well plate (white with clear bottom) | Corning | 3707 | RT |
| TMC353121 | MedChem Express | HY11097 | -20°C |

### Cytopathic effect (CPE) Assay Experimental procedure:

RSV titer: 6.9x10⁶ pfu/ml
MOI = pfu used for infection/ cell number
   1) HEp-2 cells were seeded in 384-well plates at 5000 cells per well in 25 µl assay medium and incubated for 24h at 37°C in 5% CO₂. Cells were seeded in each well expect for the top row, which contains medium only (low control).
   2) Compounds were dispensed using the Echo550 Liquid Handler. All compounds were used at 10 mM stock concentration. The positive control Ribavirin was included as control compound in all assays.
   3) Cells were expected to double once over 24h.
   4) The cells were infected the next day at RSV MOI 0.5, adding 5 µl virus diluted in assay medium.
   5) Non-infected cells were treated with 5 µl assay medium (high control)
   6) Plates were centrifuged at 400 rpm, 10 sec to allow the virus to sink onto the cells.
   7) Cells were incubated at 37°C in 5% CO₂ for 3 days.
   8) 10 µl CellTiter-Glo^{®} (Promega) was added to all wells.
   9) The plate was incubated at RT for 30min.
   10) Luminescence was measured on the Envision plate reader.

### Results

The results on infected and not infected cells are represented in Figures 1-23A and Table 1 below.

**Table 1:**

| **Compound** | **IC50 (µM)** |
|---|---|
| *Compound of the invention* | |
| **Compound #21** | **0.00015** |

| *Comparative compound* | |
|---|---|
| Compound #1 | 0.001 |
| Compound #2 | 0.001 |
| Compound #3 | 3.4 |
| Compound #4 | 0.007 |
| Compound #5 | 2 |
| Compound #6 | 0.2 |
| Compound #7 | 3.9 |
| Compound #8 | 0.1 |
| Compound #9 | 2 |
| Compound #10 | 0.005 |
| Compound #11 | 0.0045 |
| Compound #12 | 0.13 |
| Compound #13 | 0.0044 |
| Compound #14 | 0.07 |
| Compound #15 | 0.0015 |
| Compound #16 | 0.35 |
| Compound #17 | Not calculable |
| Compound #18 | 0.008 |
| Compound #19 | 0.04 |
| Compound #20 | 0.13 |
| Ribavirin | 15 |
| Presatovir | 0.0011 |

The results surprisingly show a higher % of viability for the compound #21 of the invention. IC50 for compound #21 (0.00015 µM) is at least around 7 times lower than IC50 of comparative compounds and, particularly around 50 times lower than IC50 of to the closest structural compound #4 (0.007 µM), thereby demonstrating an improved antiviral effect against HRSV for the compound #21.

### In vivo data:

An engineered recombinant HRSV expressing the firefly luciferase was used in a mouse model of infection described in Rameix-Welti et al., Nature Communications, 2014. BALB/C mice were infected with 50x10⁴ p.f.u. of virus and treated intranasally by 1mpk compound #21 or vehicle (10 mice/group). Bioluminescence was measured by intranasal injection of 50 µl of D-luciferin (200 mM) and capture of photon emission from the chest at 5 days post infection using the IVIS system.

Dorsal and ventral views of mice representative of the results are shown at Figure 24A. Comparative bioluminescence between compound #21 and mock-treated mice 5 days post infection are shown at Figure 24B. Each data point represents mean±s.e.m. Brown-Forsythe Test and Welch ANOVA followed by Dunnett's multiple comparison test; * p<0.05.

Treatment with compound #21 significantly reduced the bioluminescent signal in the lung of treated mice. The extent of inhibition of HRSV replication is largely underestimated since the bioluminescent signal was close to the lower limit of detection. Compound #21 is a highly potent anti-HRSV drug.

## Claims

1. A compound having the following formula (I): 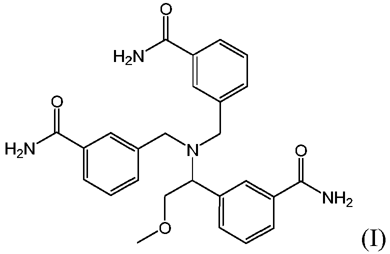 and the stereoisomers and the pharmaceutical salts thereof.

2. The compound according to claim 1, wherein said compound has the following formula (IA):

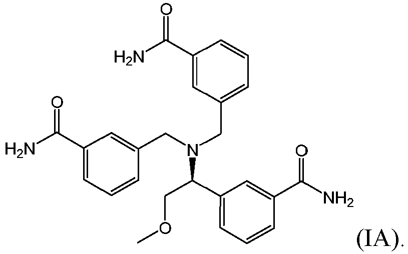

3. The compound according to claim 1, wherein said compound has the following formula (IB):

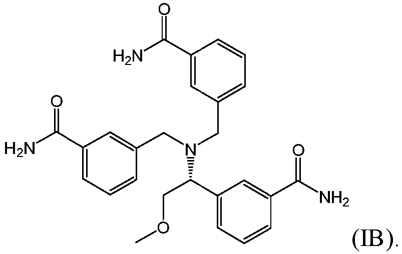

4. A compound according to any one of claims 1 to 3, for use as a medicine.

5. A pharmaceutical composition comprising a compound as defined in any one of claims 1 to 3, and an acceptable pharmaceutically excipient.

6. A pharmaceutical composition according to claim 5, for use for treating a viral infection.

7. A pharmaceutical composition for use according to claim 6, wherein the viral infection is a viral respiratory infection.

8. A pharmaceutical composition for use according to claim 7, wherein the viral respiratory infection is caused by Human Respiratory Syncytial Virus.

## Patentansprüche

1. Eine Verbindung, die die folgende Formel (I) hat: 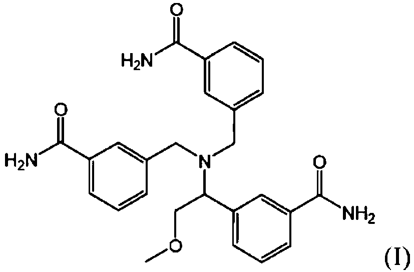 und die Stereoisomere und die pharmazeutischen Salze davon.

2. Die Verbindung nach Anspruch 1, wobei diese Verbindung die folgende Formel (IA) hat:

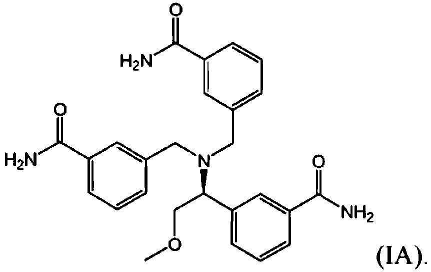

3. Die Verbindung nach Anspruch 1, wobei diese Verbindung die folgende Formel (IB) hat:

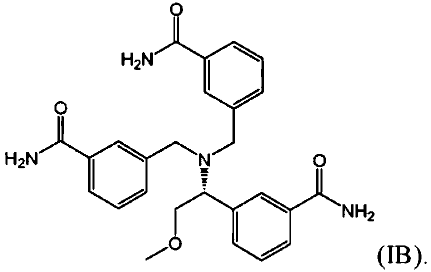

4. Eine Verbindung nach einem der Ansprüche 1 bis 3 zur Verwendung als Arzneimittel.

5. Eine pharmazeutische Zusammensetzung, umfassend eine Verbindung nach einem der Ansprüche 1 bis 3 und einen geeigneten pharmazeutischen Hilfsstoff.

6. Eine pharmazeutische Zusammensetzung nach Anspruch 5 zur Verwendung in der Behandlung einer Virusinfektion.

7. Eine pharmazeutische Zusammensetzung zur Verwendung nach Anspruch 6, wobei es sich bei der Virusinfektion um eine virale Atemwegsinfektion handelt.

8. Eine pharmazeutische Zusammensetzung zur Verwendung nach Anspruch 7, wobei die virale Atemwegsinfektion durch das Humane Respiratorische Synzytialvirus (HRSV) verursacht wird.

## Revendications

1. Composé de formule (I) suivante : 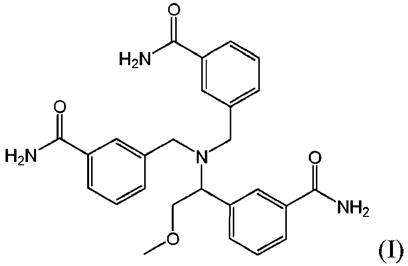 et ses stéréoisomères et sels pharmaceutiquement acceptables.

2. Composé selon la revendication 1, lequel composé est de formule (IA) suivante :

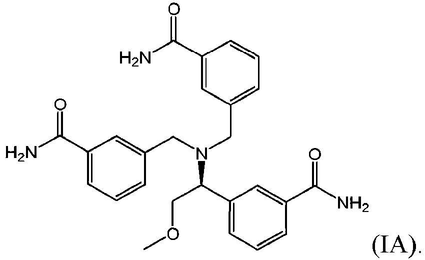

3. Composé selon la revendication 1, lequel composé est de formule (IB) suivante :

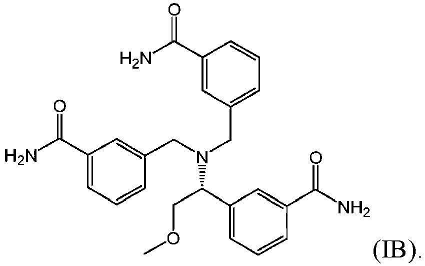

4. Composé selon l'une quelconque des revendications 1 à 3, pour une utilisation en tant que médicament.

5. Composition pharmaceutique comprenant un composé tel que défini dans l'une quelconque des revendications 1 à 3 et un excipient pharmaceutiquement acceptable.

6. Composition pharmaceutique selon la revendication 5, pour une utilisation dans le traitement d'une infection virale.

7. Composition pharmaceutique pour une utilisation selon la revendication 6, dans laquelle l'infection virale est une infection respiratoire virale.

8. Composition pharmaceutique pour une utilisation selon la revendication 7, dans laquelle l'infection respiratoire virale est causée par le virus respiratoire syncytial humain.
